# DEMANDE DE BREVET EUROPEEN

(11) **EP 1 707 241 A1**
(43) Date de publication de la demande: **04.10.2006**
(21) Numéro de dépôt: 06290507.0
(22) Date de dépôt: 31.03.2006
(51) Int. Cl.: A61Q 5/06, A61K 8/44, A61K 8/40

(54) **Composition comprenant un monomère électrophile et un acide aminé différent de la cystéine et traitement cosmétique**

(30) Priorité: 31.03.2005 FR 0503152
(71) Demandeur: L'ORÉAL, 75008 Paris (FR)
(72) Inventeur: Plos, Grégory, Tokyo 158-0097 (JP); Brun, Gaëlle, 75015 Paris (FR)
(74) Mandataire: Zapalowicz, Francis

(57) **Abrégé**

La présente invention concerne une composition fluide comprenant dans un milieu cosmétiquement acceptable, au moins un monomère électrophile et au moins un acide aminé différent de la cystéine.

L'invention est aussi relative à un procédé de traitement mettant en oeuvre cette composition et à son utilisation pour conférer un effet de conditionnement et/ou un effet coiffant aux fibres kératiniques.

## Description

La présente invention est relative à une composition comprenant dans un milieu cosmétiquement acceptable, au moins un monomère électrophile et au moins un acide aminé différent de la cystéine, à son utilisation pour le traitement cosmétique des fibres kératiniques et à un procédé de traitement cosmétique mettant en oeuvre une telle composition.

Dans le domaine de la cosmétique, on cherche à modifier les propriétés superficielles des fibres kératiniques telles que les cheveux, par exemple pour apporter aux cheveux un effet conditionnant comme la douceur, ou de la brillance. Pour ce faire, on utilise généralement des compositions cosmétiques à base d'agents de conditionnement tels que des silicones ou des polymères ayant une forte affinité pour les fibres kératiniques, et notamment pour les cheveux.

Cependant, ces agents de conditionnement ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables.

La Demanderesse a trouvé de manière surprenante qu'en associant au moins un acide aminé différent de la cystéine à au moins un monomère électrophile tel que décrit ci-dessous dans un milieu cosmétiquement acceptable, il était possible d'obtenir un conditionnement et une brillance améliorés des cheveux, et ce de manière durable.

En effet, l'application d'une composition comprenant une telle association conduit à la formation d'un revêtement ou gainage brillant rémanent notamment aux shampoings, même après plusieurs lavages de la chevelure. Le gainage obtenu peut apporter, en plus des propriétés de brillance et de conditionnement, des propriétés marquées de coiffage rémanent.

En outre, les cheveux restent de manière surprenante parfaitement individualisés et peuvent être coiffés sans problème.

L'invention a donc pour objet une composition fluide comprenant dans un milieu cosmétiquement acceptable, au moins un monomère électrophile tel que décrit ci-dessous et au moins un acide aminé différent de la cystéine.

Un autre objet de la présente invention consiste en l'utilisation de ladite composition pour le traitement cosmétique des fibres kératiniques, et plus particulièrement des cheveux.

L'invention a encore pour objet un procédé de traitement cosmétique des fibres kératiniques, et plus particulièrement des cheveux, mettant en oeuvre ladite composition.

D'autres objets, caractéristiques, aspects et avantages de l'invention apparaîtront encore plus clairement à la lecture de la description et de l'exemple qui suit.

Selon l'invention, la composition fluide comprend dans un milieu cosmétiquement acceptable, au moins un monomère électrophile et au moins un acide aminé différent de la cystéine, ledit monomère électrophile correspondant à la formule générale suivante (A) : 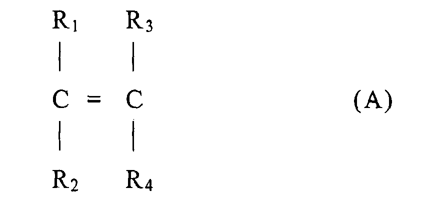 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attracteur) choisi de préférence parmi les groupements -N(R)₃⁺, -S(R)₂⁺, -SH₂⁺, -NH₃⁺, -NO₂, -SO₂R, -C≡N, - COOH, -COOR, -COSR, -CONH₂, -CONHR, -F, -Cl, -Br, -I, -OR, - COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène et un résidu de polymère pouvant être obtenu par polymérisation radicalaire ou par polycondensation ou par ouverture de cycle, R' désignant un groupe alkyle en C₁-C₁₀.

Par composition fluide, on entend au sens de la présente invention, une composition présentant une viscosité inférieure à 5 Pa.s (50 Poises), mesurée à 25 °C et à un taux de cisaillement de 1 s⁻¹, avec un viscosimètre RHEOMAT 180 de la société METTLER ou avec un viscosimètre RS600 de la société THERMO.

De préférence, la viscosité est comprise dans l'intervalle allant de 0,01 à 5 Pa.s (10 centiPoises à 50 Poises), mieux encore de 0,1 à 4 Pa.s (100 centiPoises à 40 Poises), et encore plus préférentiellement de 0,2 à 2 Pa.s (2 à 20 Poises).

Par milieu cosmétiquement acceptable, on entend un milieu compatible avec les fibres kératiniques telles que les cheveux.

De préférence le milieu cosmétiquement acceptable est anhydre.

On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique et l'alcool béhénylique, les acides gras en C₁₀-C₃₀ tels que l'acide laurique et l'acide stéarique, les amides gras en C₁₀-C₃₀ tels que le diéthanolamide laurique, les esters d'alcools gras en C₁₀-C₃₀ tels que les benzoates d'alcool gras en C₁₀-C₃₀ et leurs mélanges.

De préférence, les composés organiques constituant le milieu sont choisis parmi les composés liquides à la température de 25°C et sous 10⁵ Pa (760mm de Hg).

Le milieu cosmétiquement acceptable anhydre utilisé de préférence est choisi parmi les huiles d'olive, de ricin, de colza, de coprah, de germes de blé, d'amande douce, d'avocat, de macadania, d'abricot, de carthame, de noix de bancoulier, de camélina, de tamanu, de citron ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions α et ω (85,3% en poids), ou leurs mélanges.

Par acide aminé, on entend au sens de la présente invention un composé qui n'est pas obtenu suite à la polycondensation d'acides aminés identiques ou différents.

Les acides aminés utilisables selon l'invention comportent au moins une fonction amine et au moins une fonction acide. La ou les fonctions acides peuvent être carboxyliques, sulfoniques, phosphoniques ou phosphoriques, et de préférence carboxyliques.

De préférence, les acides aminés utilisés dans la présente invention sont des α-acides aminés, c'est-à-dire qu'ils comportent une fonction amine et un groupement R situés en position alpha par rapport à la fonction acide. Ils peuvent être représentés par la formule suivante : 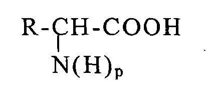 dans laquelle ;
lorsque p=2, R représente un atome d'hydrogène, un groupement aliphatique comportant ou non une portion hétérocyclique, ou un groupement aromatique,
   ou
lorsque p=1, R peut former avec l'atome d'azote de -N(H)p un hétérocycle. Cet hétérocycle est de préférence un cycle saturé à 5 chaînons, éventuellement substitué par un ou plusieurs groupements alkyle en C₁₋₄, ou hydroxy.

De préférence, le groupement aliphatique est un groupe alkyle en C₁₋₄, linéaire ou ramifié ; un groupe hydroxyalkyle en C₁₋₄, linéaire ou ramifié ; un groupe aminoalkyle en C₁₋₄, linéaire ou ramifié ; un groupe (alkyl en C₁₋₄)thioalkyle en C₁₋₄, linéaire ou ramifié ; un groupe carboxyalkyle en C₂₋₄, linéaire ou ramifié ; un groupe uréidoalkyle linéaire ou ramifié, un groupe guanidinoalkyle linéaire ou ramifié, un groupe imidazoloalkyle linéaire ou ramifié, un groupe indoylalkyle linéaire ou ramifié, les portions alkyle de ces quatre derniers groupes comportant de un à quatre atomes de carbone.

De préférence, le groupement aromatique est un groupe aryle en C₆ ou aralkyle en C₇₋₁₀, le noyau aromatique étant éventuellement substitué par un ou plusieurs groupes alkyle en C₁₋₄, ou hydroxy.

A titre d'acides aminés utilisables dans la présente invention, on peut notamment citer l'acide aspartique, l'acide glutamique, l'alanine, l'arginine, l'asparagine, la glutamine, la glycine, l'histidine, l'isoleucine, la leucine, la lysine, la méthionine, la N-phénylalanine, la proline, la serine, la thréonine, le tryptophane, la tyrosine, la valine et l'hydroxyproline.

De manière préférentielle, les acides aminés ne comportent qu'une seule fonction amine engagée ou non dans un cycle, et une seule fonction acide. Les acides aminés particulièrement préférés dans la présente invention sont l'alanine, l'asparagine, la glutamine, la glycine, l'isoleucine, la leucine, la méthionine, la N-phénylalanine, la proline, la serine, la thréonine, la tyrosine et la valine.

Les compositions mises en oeuvre conformément à l'invention ont généralement une concentration d'acide(s) aminé(s) comprise entre 0,1 et 20 % en poids, de préférence entre 0,5 et 15 % en poids, plus particulièrement entre 1 et 10 % en poids par rapport au poids total de la composition.

Par monomère électrophile, on entend un monomère capable de polymériser par polymérisation anionique en présence d'un agent nucléophile tel que par exemple, les ions hydroxydes (OH⁻) contenus dans l'eau à pH neutre.

Par polymérisation anionique, on entend le mécanisme défini dans l'ouvrage "Advanced Organic Chemistry", Third Edition de Jerry March, pages 151 à 161.

Les monomères électrophiles utilisés conformément à l'invention sont les monomères de structure : 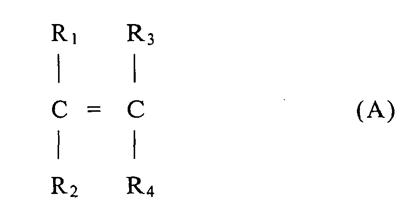 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attracteur) choisi de préférence parmi les groupements -N(R)₃⁺, -S(R)₂⁺, -SH₂⁺, -NH₃⁺, -NO₂ -SO₂R, -C≡N, - COOH, -COOR, -COSR, -CONH₂, -CONHR, -F, -Cl, -Br, -I, -OR, - COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène et un résidu de polymère pouvant être obtenu par polymérisation radicalaire ou par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6, 111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou - (CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (B) : 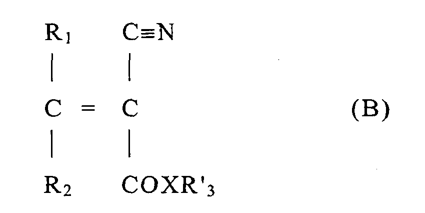 X désignant NH,S,O,
R₁ et R₂ ayant les mêmes significations que précédemment,
R'₃ pouvant désigner un atome d'hydrogène ou un groupe R tel que défini pour la formule (A).

De préférence, X désigne O.

A titre de composés de formule (B), on peut citer les monomères :
a) appartenant à la famille des 2-cyanoacrylates de polyfluoroalkyle en C₁₋₂₀ tels que :
   l'ester 2,2,3,3-tétrafluoropropylique de l'acide 2-cyano-2-propénoïque de formule : 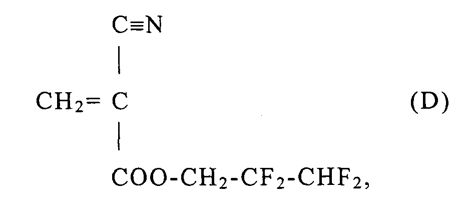
   ou encore l'ester 2,2,2-trifluoroéthylique de l'acide 2-cyano-2-propénoïque de formule : 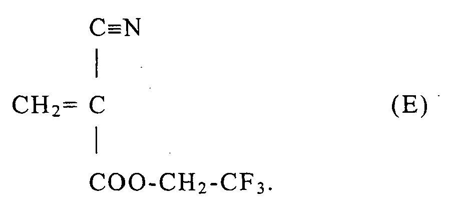
b) les cyanoacrylates d'alkyle en C₁-C₁₀ ou d'(alcoxy en C₁₋ C₄)(alkyle en C₁-C₁₀).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères les plus particulièrement préférés sont ceux de formule F et leurs mélanges : 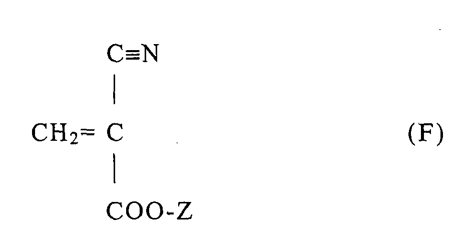 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Les compositions mises en oeuvre conformément à l'invention ont généralement une concentration en monomère électrophile selon l'invention comprise entre 0,001 et 80 % en poids, de préférence entre 0,002 et 75 % en poids, plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène-ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur(s) peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des propulseurs, et des épaississants minéraux ou organiques tels que le benzylidène-sorbitol et les N-acylaminoacides.

Ces agents ou le(s) acide(s) aminé(s) peuvent être éventuellement encapsulés. La capsule peut être de type polycyanoacrylate.

La composition est utilisée de préférence sur les fibres kératiniques comme les cheveux, de préférence en présence d'un agent nucléophile, pour leur traitement cosmétique.

Elle est notamment utilisée pour conférer un effet de conditionnement tel que de la douceur ; du démêlage; de la brillance ; du volume et/ou un effet coiffant comme le maintien de la chevelure.

Un procédé de traitement cosmétique selon l'invention comprend l'application d'une composition telle que définie ci-dessus, sur les fibres kératiniques comme les cheveux, de préférence en présence d'un agent nucléophile tel que défini ci-dessous.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable.

Il est également possible, afin de moduler la cinétique de réaction, de préalablement humidifier les fibres kératiniques comme les cheveux, à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les fibres kératiniques comme les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des fibres kératiniques comme les cheveux, par transformation chimique de la matière kératinique.

A titre d'exemple de transformation chimique, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Elles peuvent se présenter sous la forme de lotion, de spray, de mousse, et être appliquées comme shampoing ou après-shampoing.

Un autre objet de l'invention consiste en un procédé de traitement cosmétique des fibres kératiniques, comprenant au moins deux étapes, une étape comprenant l'application d'au moins un acide aminé différent de la cystéine tel que défini ci-dessus, et, avec ou sans rinçage intermédiaire, une autre étape comprenant l'application d'au moins un monomère électrophile tel que défini ci-dessus, l'ordre des étapes étant indifférent.

Un mode de réalisation particulier de l'invention consiste en ce que l'application d'au moins un acide aminé différent de la cystéine se fait avant l'application d'au moins un monomère électrophile.

Un autre mode de réalisation particulier de l'invention consiste en ce que l'application d'au moins un monomère électrophile se fait avant l'application d'au moins un acide aminé différent de la cystéine.

Un mode de réalisation préféré de l'invention consiste en un procédé comprenant les étapes consistant à :
(1) appliquer sur les cheveux une solution aqueuse à 0,05-40 % en poids, de préférence 0,1-35 % en poids, et mieux encore 0,25-25 % en poids d'acide(s) aminé(s), les pourcentages étant exprimés par rapport au poids total de la solution,
(2) appliquer sur les cheveux, après un éventuel rinçage intermédiaire, une composition comprenant 0,001-80 % en poids, de préférence 0,1 à 40 % en poids, et mieux encore 1 à 20 % en poids de monomère(s) électrophile(s) tel(s) que défini(s) ci-dessus, les pourcentages étant exprimés par rapport au poids total de la composition.

L'ordre des deux étapes (1) et (2) peut être inversé.

L'application d'un produit cosmétique peut en outre précéder la première étape ou suivre la deuxième étape. Chaque étape peut être également interrompue par un rinçage et éventuellement par un séchage, le séchage pouvant être réalisé au moyen d'un casque, d'un sèche-cheveux et/ou d'un fer à lisser.

Les étapes des procédés décrits ci-dessus peuvent être répétées de manière à obtenir plusieurs couches selon le type de dépôt que l'on recherche en termes de nature chimique, résistance mécanique, épaisseur, aspect et/ou toucher.

L'invention a encore pour objet un kit comprenant une première composition contenant au moins un monomère électrophile tel que défini ci-dessus et éventuellement au moins un inhibiteur de polymérisation anionique et/ou radicalaire tel que défini ci-dessus, ainsi qu'une deuxième composition comprenant dans un milieu cosmétiquement acceptable au moins un acide aminé différent de la cystéine tel que défini ci-dessus.

L'exemple suivant est donné à titre illustratif de la présente invention.

Dans l'exemple suivant, toutes les quantités sont indiquées en pour cent en poids de matière active par rapport au poids total de la composition, sauf indication contraire.

### EXEMPLE

On a préparé une composition A comprenant les ingrédients suivants, les proportions étant indiquées en % en poids.

| | |
|---|---|
| - 2-cyanoacrylate de 2-octyle | 10 % |
| - Mélange 50/50 en poids de DC 1501 Fluid (Dow Corning) (poly(alpha-omega dihydroxyl- diméthylsiloxane/ cyclopentadiméthylsiloxane) (14,7/85,3)) et de DC 245 Fluid (Dow Corning) (cyclopentadiméthylsiloxane) | 90 % |

On a humidifié une mèche de 1 g. de cheveux naturels châtains, avec 0,5 g d'une solution aqueuse à 0,2 mol/l de glycine.

On a ensuite appliqué 0,16 g de la composition A sur la mèche.

Après 15 minutes de pose, on a séché la mèche pendant 2 minutes au sèche-cheveux. La mèche obtenue est plus brillante qu'une mèche qui a été humidifiée avec 0,5 g d'eau à la place de la solution de glycine.

## Revendications

1. Composition fluide comprenant dans un milieu cosmétiquement acceptable, au moins un monomère électrophile et au moins un acide aminé différent de la cystéine, ledit monomère électrophile correspondant à la formule générale suivante (A) : 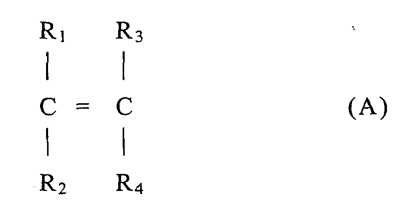 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR, -COOR, -COR, - SH, -SR, -OH et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, -S(R)₂⁺, - SH₂⁺, -NH₃⁺, -NO₂, -SO₂R, -C≡N, -COOH, -COOR, -COSR, -CONH₂,-CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle et aryloxy ;
R désigne un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR', -COOR', -COR', -SH, -SR', -OH, les atomes d'halogène, et un résidu de polymère pouvant être obtenu par polymérisation radicalaire ou par polycondensation ou par ouverture de cycle, R' désignant un groupe alkyle en C₁-C₁₀.

2. Composition selon la revendication 1, **caractérisée en ce que** l'acide aminé comporte une fonction acide carboxylique.

3. Composition selon la revendication 1 ou 2, **caractérisée en ce que** l'acide aminé est un α-acide aminé.

4. Composition selon l'une quelconque des revendications précédentes, **caractérisée en ce que** l'acide aminé répond à la formule : 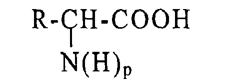 dans laquelle :
lorsque p=2, R représente un atome d'hydrogène, un groupement aliphatique comportant ou non une portion hétérocyclique, ou un groupement aromatique,
ou
lorsque p=1, R peut former avec l'atome d'azote de -N(H)ₚ un hétérocycle.

5. Composition selon la revendication 4, **caractérisée en ce que** le groupement aliphatique est un groupe alkyle en C₁₋₄, linéaire ou ramifié ; un groupe hydroxyalkyle en C₁₋₄, linéaire ou ramifié ; un groupe aminoalkyle en C₁₋₄, linéaire ou ramifié ; un groupe (alkyl en C₁₋₄)thioalkyle en C₁₋₄, linéaire ou ramifié ; un groupe carboxyalkyle en C₂₋₄, linéaire ou ramifié ; ou un groupe uréidoalkyle linéaire ou ramifié, un groupe guanidinoalkyle linéaire ou ramifié, un groupe imidazoloalkyle linéaire ou ramifié, un groupe indoylalkyle linéaire ou ramifié, les portions alkyle de ces quatre derniers groupes comportant de un à quatre atomes de carbone.

6. Composition selon la revendication 4, **caractérisée en ce que** le groupement aromatique est un groupe aryle en C₆ ou aralkyle en C₇₋₁₀, le noyau aromatique étant éventuellement substitué par un ou plusieurs groupes alkyle en C₁₋₄, ou hydroxy.

7. Composition selon la revendication 4, **caractérisée en ce que** l'hétérocycle, lorsque p=1, est un cycle saturé à 5 chaînons, éventuellement substitué par un ou plusieurs groupements alkyle en C₁₋₄, ou hydroxy.

8. Composition selon l'une quelconque des revendications précédentes, **caractérisée en ce que** ledit acide aminé est choisi parmi l'acide aspartique, l'acide glutamique, l'alanine, l'arginine, l'asparagine, la glutamine, la glycine, l'histidine, l'isoleucine, la leucine, la lysine, la méthionine, la N-phénylalanine, la proline, la serine, la thréonine, le tryptophane, la tyrosine, la valine et l'hydroxyproline.

9. Composition selon la revendication 8, **caractérisée en ce que** ledit acide aminé est choisi parmi l'alanine, l'asparagine, la glutamine, la glycine, l'isoleucine, la leucine, la méthionine, la N-phénylalanine, la proline, la serine, la thréonine, la tyrosine et la valine.

10. Composition selon l'une quelconque des revendications précédentes, **caractérisée en ce que** ledit ou lesdits acides aminés sont présents en une concentration comprise entre 0,1 et 20 % en poids, de préférence entre 0,5 et 15 % en poids, par rapport au poids total de la composition.

11. Composition selon la revendication 1, **caractérisée en ce que** le ou les monomères électrophiles sont choisis parmi les composés de formule : 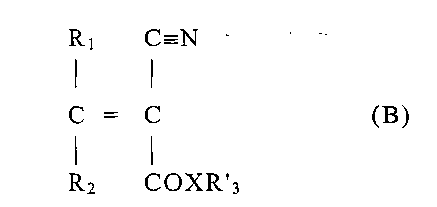 X désignant NH,S,O,
R₁ et R₂ sont tels que définis dans la revendication 1,
R'₃ pouvant désigner un atome d'hydrogène ou un groupe R tel que défini dans la revendication 1.

12. Composition selon la revendication 11, **caractérisée par le fait que** le ou les monomères sont choisis parmi les 2-cyanoacrylates de polyfluoroalkyle en C₁₋₂₀, les cyanoacrylates d'alkyle en (C₁-C₁₀) ou d'(alcoxy en C₁-C₄)(alkyle en C₁-C₁₀).

13. Composition selon la revendication 12, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

14. Composition selon la revendication 13, **caractérisée en ce que** le ou les monomères électrophiles répondent à la formule (F) : 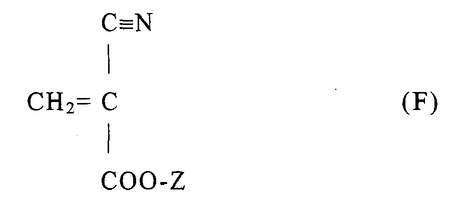 dans laquelle : Z=-(CH₂)₇-CH₃,
-CH(CH₃)-(CH₂)₅-CH₃,
-CH₂-CH(C₂H₅)-(CH₂)₃₋CH₃,
-(CH₂)₅-CH(CH₃)-CH₃,
-(CH₂)₄-CH(C₂H₅)-CH₃.

15. Composition selon l'une quelconque des revendications précédentes, **caractérisée en ce que** le ou les monomères électrophiles sont fixés de façon covalente sur des supports tels que des polymères, des oligomères ou des dendrimères.

16. Composition selon l'une quelconque des revendications précédentes, **caractérisée en ce que** la quantité de monomère électrophile va de 0,001 à 80 % en poids, de préférence de 0,002 à 75 % en poids, encore plus préférentiellement de 0,1 à 40 % en poids par rapport au poids total de la composition.

17. Composition selon l'une quelconque des revendications précédentes, **caractérisée en ce que** le milieu cosmétiquement acceptable est anhydre.

18. Composition selon la revendication 17, **caractérisée en ce que** le milieu cosmétiquement acceptable est choisi est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

19. Composition selon la revendication 18, **caractérisée en ce que** le milieu cosmétiquement acceptable comprend est choisi parmi les huiles d'olive, de ricin, de colza, de coprah, de germes de blé, d'amande douce, d'avocat, de macadania, d'abricot, de carthame, de noix de bancoulier, de camélina, de tamanu, de citron ; l'huile de polybutène, l'isononanoate d'isononyle, le malate d'isostéartyle, le tétra-isostéarate de pentaérythrityle, le trimélate de tridécyle, le mélange cyclopentasiloxane/polydiméthylsiloxane dihydroxylé en positions α et ω, ou leurs mélanges.

20. Composition selon l'une quelconque des revendications précédentes, **caractérisée en ce que** la composition contient des inhibiteurs de polymérisation.

21. Composition selon la revendication 20, **caractérisée en ce que** les inhibiteurs sont des inhibiteurs de polymérisation anioniques et/ou radicalaires.

22. Composition selon la revendication 20 ou 21, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

23. Composition selon l'une quelconque des revendications 20 à 22, **caractérisée en ce que** le ou les inhibiteurs de polymérisation sont présents en une quantité allant de 10 ppm à 20 % par rapport au poids total de la composition.

24. Composition selon l'une quelconque des revendications précédentes, **caractérisée en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, des vitamines, les colorants directs ou d'oxydation, les agents nacrants, les propulseurs et les agents épaississants minéraux ou organiques.

25. Composition selon la revendication 24, **caractérisée en ce que** l'agent est encapsulé.

26. Utilisation de la composition selon l'une quelconque des revendications précédentes, pour le traitement cosmétique des fibres kératiniques.

27. Utilisation selon la revendication 26, pour conférer de la douceur, du démêlage, de la brillance, du volume et/ou du maintien aux cheveux.

28. Utilisation selon la revendication 26 ou 27, en présence d'un agent nucléophile.

29. Utilisation selon la revendication 28, **caractérisée en ce que** l'agent nucléophile est choisi parmi les composés moléculaires, les oligomères, les dendrimères ou polymères possédant des fonctions nucléophiles choisies parmi : R₂N⁻, NH₂', Ph₃C⁻, R₃C⁻, PHNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

30. Utilisation selon la revendication 29, **caractérisée en ce que** l'agent nucléophile est l'eau.

31. Procédé de traitement cosmétique des fibres kératiniques, comprenant l'application d'une composition selon l'une quelconque des revendications précédentes 1 à 25, sur les fibres kératiniques en présence d'un agent nucléophile.

32. Procédé selon la revendication 31, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PHNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN', NO₃-, ClO₄⁻ et H₂O Ph représentant le groupe phényle, Ar représentant un groupe aryle et R représentant un groupe aryle en C₁-C₁₀.

33. Procédé selon la revendication 32, **caractérisé en ce que** l'agent nucléophile est l'eau.

34. Procédé selon l'une quelconque des revendications 31 à 33, **caractérisé en ce que** l'on applique la composition sur les fibres kératiniques préalablement humidifiées à l'aide d'une solution aqueuse dont le pH a été ajusté à l'aide d'une base, d'un acide ou d'un mélange acide/base.

35. Procédé selon l'une quelconque des revendications 31 à 33, **caractérisé en ce que** les fibres kératiniques sont pré-imprégnées à l'aide d'un agent nucléophile différent de l'eau.

36. Procédé selon l'une quelconque des revendications 31 à 33, **caractérisé en ce que** les fibres kératiniques sont préalablement réduites avant application de la composition.

37. Procédé selon l'une des revendications 31 à 36, **caractérisé en ce que** l'application de la composition est suivie d'un rinçage.

38. Procédé selon l'une des revendications 31 à 37, **caractérisé en ce que** les fibres kératiniques sont les cheveux.

39. Procédé de traitement cosmétique des fibres kératiniques, comprenant au moins deux étapes, une étape comprenant l'application d'au moins un acide aminé différent de la cystéine tel que défini dans l'une quelconque des revendications 2 à 9, et avec ou sans rinçage intermédiaire, une autre étape comprenant l'application d'au moins un monomère électrophile tel que défini dans l'une quelconque des revendications 1 et 11 à 14.

40. Procédé selon la revendication 39, **caractérisé en ce que** l'application d'au moins un acide aminé différent de la cystéine se fait avant l'application d'au moins un monomère électrophile.

41. Procédé selon la revendication 39, **caractérisé en ce que** l'application d'au moins un monomère électrophile se fait avant l'application d'au moins un acide aminé différent de la cystéine.

42. Procédé selon la revendication 40, **caractérisé en ce qu'**il comprend les étapes consistant à :
(1) appliquer sur les cheveux une solution aqueuse à 0,05-40 % en poids, de préférence 0,1-35 % en poids, en poids d'acide(s) aminé(s), les pourcentages étant exprimés par rapport au poids total de la solution,
(2) appliquer sur les cheveux, après un éventuel rinçage intermédiaire, une composition comprenant 0,001-80 % en poids, de préférence 0,1 à 40 % en poids, de monomère(s) électrophile(s) tel(s) que défini(s) ci-dessus, les pourcentages étant exprimés par rapport au poids total de la composition.

43. Kit comprenant une première composition contenant au moins un monomère électrophile tel que défini dans l'une quelconque des revendications 1 et 11 à 14, et éventuellement au moins un inhibiteur de polymérisation anionique et/ou radicalaire, ainsi qu'une deuxième composition comprenant dans un milieu cosmétiquement acceptable au moins un acide aminé différent de la cystéine tel que défini dans l'une quelconque des revendications 2 à 9.
